Europäisches Patentamt

European Patent Office

Office européen des brevets

(19)

(11) Publication number: **0 111 389**
**A2**

(12)

# EUROPEAN PATENT APPLICATION

(21) Application number: **83306730.9**

(22) Date of filing: **04.11.83**

(51) Int. Cl.³: **C 12 N 15/00**
C 12 P 21/02, C 12 N 1/20
C 12 N 5/00
//C12R1/19, C12R1/91

(30) Priority: **08.11.82 US 439977**

(43) Date of publication of application:
**20.06.84 Bulletin 84/25**

(84) Designated Contracting States:
**AT BE CH DE FR GB IT LI LU NL SE**

(71) Applicant: **GENENTECH, INC.**
**460 Point San Bruno Boulevard**
**So. San Francisco California 94080(US)**

(72) Inventor: **Adelman, John P.**
**1028 Escalero**
**Pacifica California 94044(US)**

(72) Inventor: **Seeburg, Peter H.**
**800 Kirkham**
**San Francisco California 94122(US)**

(74) Representative: **Armitage, Ian Michael et al,**
**MEWBURN ELLIS & CO. 2/3 Cursitor Street**
**London EC4A 1BQ(GB)**

(54) **Substantially pure porcine growth hormone, DNA sequences therefor, and expression vehicles and transfected microorganisms for the production of porcine growth hormone.**

(57) The DNA and amino acid sequence of porcine growth hormone are disclosed. The DNA is used to produce substantially pure porcine growth hormone by recombinant DNA technology.

EP 0 111 389 A2

Croydon Printing Company Ltd.

DOCKET:  100/78

## SUBSTANTIALLY PURE PORCINE GROWTH HORMONE, DNA SEQUENCES THEREFOR, AND EXPRESSION VEHICLES AND TRANSFECTED MICROORGANISMS FOR THE PRODUCTION OF PORCINE GROWTH HORMONE

### Field of the Invention

The present invention is generally related to recombinant DNA technology.  It is based upon advances utilizing such technology for the preparation of a hitherto unknown DNA sequence encoding a hitherto unknown amino acid sequence of a basic animal protein, porcine growth hormone.  The present invention is based upon the discovery of the DNA sequence of said protein and to the deduced, complete amino acid sequence thereof as well as to the exploitation of such information for the high level production of porcine growth hormone from genetically altered cell cultures harboring recombinant DNA sequences capable of effecting expression in the cell culture.

### Background of the Invention

Means and methods for the production by genetically altered cell cultures of numerous polypeptides, including human growth hormone, were disclosed by Goeddel et al. in U.S.S.N. 55126, filed July 5, 1979, now U.S. Patent No. 4342832, issued August 3, 1982, which is hereby incorporated by this reference.  Likewise, the production of certain human growth hormone variants has been disclosed in U.S.S.N. 06/360,517, filed March 22, 1982, also incorporated herein by reference.  In addition, bovine growth hormone has been produced in

0200L

cell culture by the expression of DNA sequence encoding it, taking advantage of the repair of certain secondary/tertiary conformational structural impediments in the messenger RNA transcripts attending the translation phase of the heterologous gene expression. See U.S.S.N. 06/303,687, filed September 18, 1981, which is also hereby incorporated by this reference.

It is noteworthy, in the present context, that the production of both of these proteins, human growth hormone and bovine growth hormone, involved the preparation of hybrid DNA sequences encoding the respective protein prior to their incorporation into an expression vector for use in transfecting a host cell which then was directed in its production of the respective desired protein. Such hybrid DNA sequences were enabled by the synthetic preparation of a portion of the 5'-end in such manner as to facilitate its proper reading frame ligation with the remainder of the DNA sequence obtained most preferably from cDNA derived from messenger RNA source tissue. The use of such hybrid genes facilitated also the proper incorporation of the gene within an expression vector so as to ensure proper reading phase operability, and in the case of bovine growth hormone, the elimination of translational inhibiting presence of secondary/tertiary conformational structure.

The present invention, directed to the production of porcine growth hormone from genetically altered cell cultures through the application of recombinant DNA technology, avoids any necessity of constructing such hybrid DNA sequences for its enablement. Furthermore, the present invention is based upon the discovery of the DNA sequence of porcine growth hormone and the consequential deduced, complete amino acid sequence for this protein as well as the consequential realization that such DNA sequence, not containing sufficiently interfering secondary/tertiary conformational structure, could be linked directly to operable promoter sequences so as to ensure its efficient production via expression of the gene by cell culture so directed.

0200L

Thus, the nature of the discovery of the DNA and the amino acid sequence for porcine growth hormone enable improved refinements in recombinant DNA techniques useful in providing yet another desired heterologous protein under such circumstances.

## Summary of the Invention

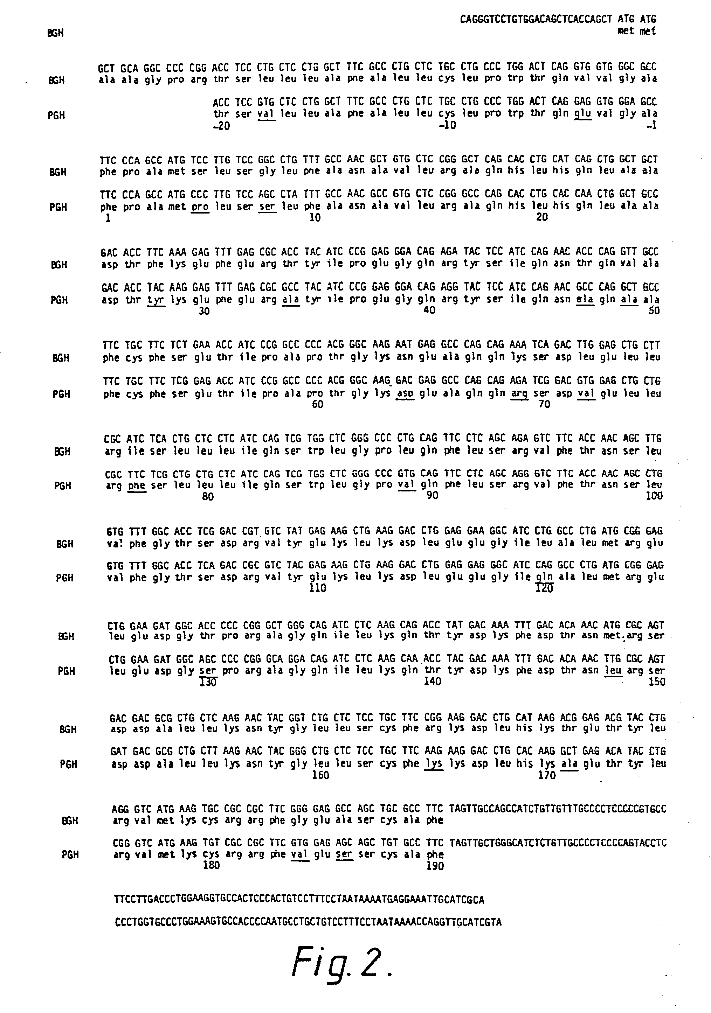

The present invention is directed, as a primary aspect, to the DNA sequence encoding porcine growth hormone, particularly as depicted in Figure 2 hereof, including nucleotide variations therein based upon the known degeneracy of the genetic code, as well as those nucleotide sequences encoding allelic versions of porcine growth hormone. The present invention is further directed, in another aspect, to the amino acid sequence of porcine growth hormone, as substantially depicted in Figure 2 hereof, including allelic versions thereof. The present invention is directed to processes for the construction of various replicable cloning vehicles harboring said DNA sequences as well as expression vehicles harboring said DNA sequences useful to direct the production of porcine growth hormone in a transfected cell culture. The porcine growth hormone of the present invention can thus be prepared in mature form, that is devoid of any presequence or other sequence as an artifact of expression, or as a fusion protein with a synthetically derived host cell culture protein or its own signal presequence or portion thereof. The present invention also encompasses the various replicable cloning vehicles, expression vehicles, and transfected cell cultures as described above, all harboring the altered genetic information necessary to effect the production of porcine growth hormone or derivatives thereof by the transfected cell culture.

The porcine growth hormone of the present invention is produced in substantially pure form -- in contrast to results obtainable by (now obsolete) extraction from natural source -- and exists, by

0200L

virtue hereof, essentially free of other proteins of porcine origin. Notwithstanding such levels of hitherto unobtainable purity, upon production, the protein can be formulated with conventional carriers and adjuvants, including other proteins, for example, serum albumin, so as to facilitate its efficacious delivery to host animal.

Notwithstanding that reference is made to particular preferred embodiments, it will be understood that the present invention encompasses the production of porcine growth hormone via genetically altered cell culture in all of its aspects and is not to be construed as limited to any such particularly preferred embodiments, rather to the lawful scope of the appended claims.

Description of Preferred Embodiments

A. Porcine Growth Hormone

The invention illustrates the production of porcine growth hormone (pGH) via genetically altered cell culture. pGH is a porcine endogenous protein and is responsible for proper physical maturation of the animal. It is largely species specific in its action and is produced by the mammalian pituitary gland. It is responsible for linear growth and is involved in the regulation of a variety of anabolic processes. As such, it is useful for increasing weight gain, feed conversion efficiency, and lean to fat ratio when administered to the animal. Until now, insufficient quantities have been available by isolation from natural source as to determine even the amino acid sequence of the protein to say nothing of providing administrable quantities for use in the animal husbandry field. The precise identity of the amino acid sequence as well as the DNA sequence for this protein, and allelic versions thereof, has now been made possible by the present invention. Consequently, the present invention enables the production of commercial quantities of the compound, further enabling now its application on a large scale in the animal husbandry field.

Discovery of the DNA sequence and amino acid sequence for porcine growth hormone has made possible the determination that the protein can be produced by a genetically altered cell culture in mature form either by use solely of cDNA or by construction of synthetic DNA, in any event arriving at a DNA sequence, encoding the proper amino acid sequence, and inserting same into an appropriate expression vector via recombinant DNA techniques for production in cell culture.

B. Microorganisms, Cell Cultures

1. Bacterial Strains/Promoters

The work described herein was performed employing, inter alia, the microorganism E. coli K-12 strain 294 (end A, thi⁻, hsr⁻, $_k$hsm⁺), as described in reference (1). This strain has been deposited with the American Type Culture Collection, ATCC Accession No. 31446, on October 28, 1978. However, various other microbial strains are useful, including known E. coli strains such as E. coli B, E. coli X 1776 (ATCC No. 31537, deposited July 3, 1979) and E. coli W 3110 (F⁻, λ⁻, protrophic) (ATCC No. 27325), or other microbial strains many of which are deposited and (potentially) available from recognized microorganism depository institutions, such as the American Type Culture Collection (ATCC)--cf. the ATCC catalogue listing. See (2). These other microorganisms include, for example, Bacilli such as Bacillus subtilis and other enterobacteriaceae among which can be mentioned as examples Salmonella typhimurium and Serratia marcesans, utilizing plasmids that can replicate and express heterologous gene sequences therein.

As examples of useful promoters, the beta lactamase and lactose promoter systems have been advantageously used to initiate and

0200L

sustain microbial production of heterologous polypeptides. Details relating to the make-up and construction of these promoter systems can be had by reference to (3,4,5). More recently, a system based upon tryptophan, the so-called trp promoter system, has been developed. Details relating to the make-up and construction of this system can be had by reference to (6) and (7). Numerous other microbial promoters have been discovered and utilized and details concerning their nucleotide sequences, enabling a skilled worker to ligate them functionally within plasmid vectors, have been published -- See (8).

   2.  Yeast Strains/Yeast Promoters

   The expression system hereof may also employ, for example, the plasmid YRp7 (9, 10, 11), which is capable of selection and replication in both E. coli and the yeast, Saccharomyces cerevisiae. For selection in yeast the plasmid contains the TRP1 gene which complements (allows for growth in the absence of tryptophan) yeast containing mutations in this gene found on chromosome IV of yeast (12). A useful strain is strain RH218 (13) deposited at the American Type Culture Collection without restriction (ATCC No. 44076). However, it will be understood that any Saccharomyces cerevisiae strain containing a mutation which makes the cell trp1 should be an effective environment for expression of the plasmid containing the expression system. An example of another strain which could be used is pep4-1 (14). This tryptophan auxotroph strain also has a point mutation in TRP1 gene.

   When placed on the 5' side of a non-yeast gene the 5'-flanking DNA sequence (promoter) from a yeast gene (for alcohol dehydrogenase 1) can promote the expression of a foreign gene in yeast when placed in a plasmid used to transform yeast. Besides a promoter, proper expression of a non-yeast gene in yeast requires a second yeast sequence placed at the 3'-end of the non-yeast gene on the plasmid so as to allow for proper transcription termination and polyadenylation in yeast. This promoter can be suitably employed in the present invention as well as others -- see infra.  In the preferred embodiments, the 5'-flanking sequence of the yeast 3-phosphoglycerate kinase gene (15) is placed upstream from the structural gene followed again by DNA containing termination - polyadenylation signals, for example, the TRP1 gene or the PGK gene.

3.  Cell Culture Systems/Cell Culture Vectors

Propogation of vertebrate cells in culture (tissue culture) has become a routine procedure in recent years.  (See 16).  A useful host for the production of heterologous protein is the COS-7 line of monkey kidney fibroblasts (17).  However, the present invention could be practiced in any cell line that is capable of the replication and expression of a compatible vector, e.g., WI38, BHK, 3T3, CHO, VERO, and HeLa cell lines.  Additionally, what is required of the expression vector is an origin of replication and a promoter located in front of the gene to be expressed, along with any necessary ribosome binding sites, RNA splice sites, polyadenylation site, and transcriptional terminator sequences.  It will be understood that this invention, although described herein in terms of a preferred embodiment, should not be construed as limited to these sequences.  For example, the origin of replication of other viral (e.g., Polyoma, Adeno, VSV, BPV, and so forth) vectors could be used, as well as cellular origins of DNA replication which could function in a nonintegrated state.

C.  Vector Systems

1.  Expression in Bacterial Systems

Expression plasmids for bacterial use, e.g., E. coli are commonly derived using BR322 (18) as vector and appropriately inserting the heterologous gene sequence together with translational start and stop signals in operable reading phase with a functional promoter, taking advantage of common or synthetically created restriction sites.  The vector will carry one or more phenotypic selection characteristic genes and an origin of replication to insure amplification within the host. Again, the heterologous insert can be aligned so as to be expressed together with a fused presequence, derivable for example from the trp system genes.

2. Expression in Yeast

To express a heterologous gene such as the cDNA for pGH in yeast, it is necessary to construct a plasmid vector containing four components. The first component is the part which allows for transformation of both E. coli and yeast and thus must contain a selectable gene from each organism. This can be the gene for ampicillin resistance from E. coli and the gene TRP1 from yeast.) This component also requires an origin of replication from both organisms to be maintained as a plasmid DNA in both organisms, for example, the E. coli origin from pBR322 and the arsl origin from chromosome III of yeast.)

The second component of the plasmid is a 5'-flanking sequence from a highly expressed yeast gene to promote transcription of a downstream-placed structural gene. The 5'-flanking sequence can be that from the yeast 3-phosphoglycerate kinase (PGK) gene. The fragment is constructed in such a way so as to remove the ATG of the PGK structural sequence, replaced with a sequence containing alternative restriction sites, such as XbaI and EcoRI restriction sites, for convenient attachment of this 5'-flanking sequence to the structural gene.

The third component of the system is a structural gene constructed in such a manner that it contains both an ATG translational start and translational stop signals. The isolation and construction of such a gene is described infra.

The fourth component is a yeast DNA sequence containing the 3'-flanking sequence of a yeast gene, which contains the proper signals for transcription termination and polyadenylation.

3. Expression in Mammalian Cell Culture

The strategy for the synthesis of heterologous peptide in mammalian cell culture relies on the development of a vector capable of both autonomous replication and expression of a foreign gene under the control of a heterologous transcriptional unit. The replication of this vector in tissue culture is accomplished by providing a DNA replication origin (such as from SV40 virus), and providing helper function (T antigen) by the introduction of the vector into a cell line endogenously expressing this antigen (19, 20). The late promoter of SV40 virus precedes the structural gene and ensures the transcription of the gene.

A useful vector to obtain expression consists of pBR322 sequences which provides a selectable marker for selection in E. coli (ampicillin resistance) as well as an E. coli origin of DNA replication. These sequences are derivable from the plasmid pML-1 (21). The SV40 origin is derivable from a 342 base pair PvuII-HindIII fragment encompassing this region (22, 23) (both ends being convertable to EcoRI ends). These sequences, in addition to comprising the viral origin of DNA replication, encode the promoter for both the early and late transcriptional unit. The orientation of the SV40 origin region is such that the promoter for the late transcriptional unit is positioned proximal to the gene encoding interferon.

Brief Descriptions of the Drawings

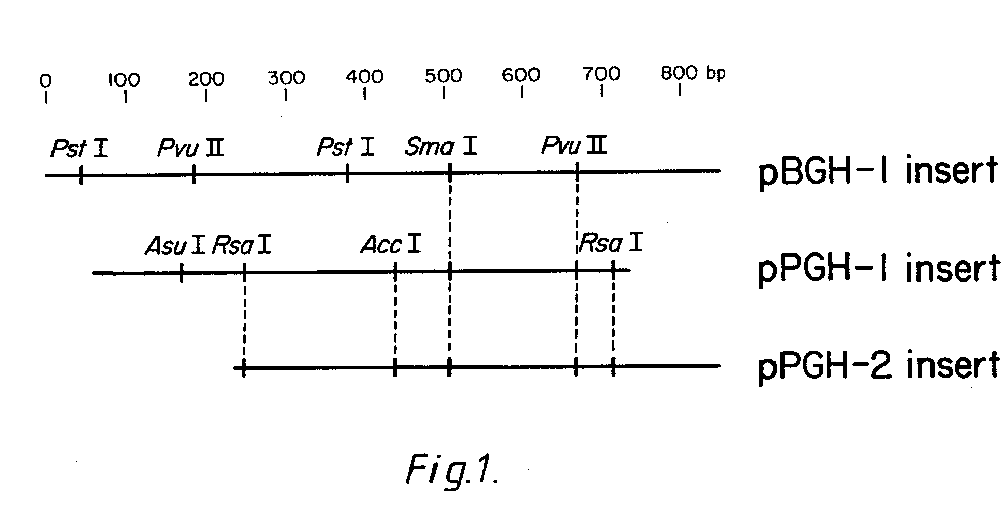

Figure 1 depicts restriction endonuclease maps of three cloned cDNAs containing coding sequences for bovine and porcine growth hormones. Dashed lines denote common cleavage sites. The AsuI site shown in one of the inserts is not unique. For coding potentials of pPGH-1 and pPGH-2, see legend to Figure 2.

Figure 2 shows the nucleotide and amino acid sequences of PGH cDNA and protein. The PGH cDNA sequence was assembled from clones pPGH-1 (starting at coding sequence for signal peptide and ending at nucleotide 40 in 3' untranslated region) and pPGH-2 (starting at codon for amino acid residue 38 and containing entire 3' noncoding region). Negative numbers refer to amino acid residues in the signal peptide and positive numbers to those in the mature hormone. The nucleotide and amino acid sequences of BGH cDNA and protein is provided for comparative purposes. (Underlined amino acid residues differ in BGH and PGH.)

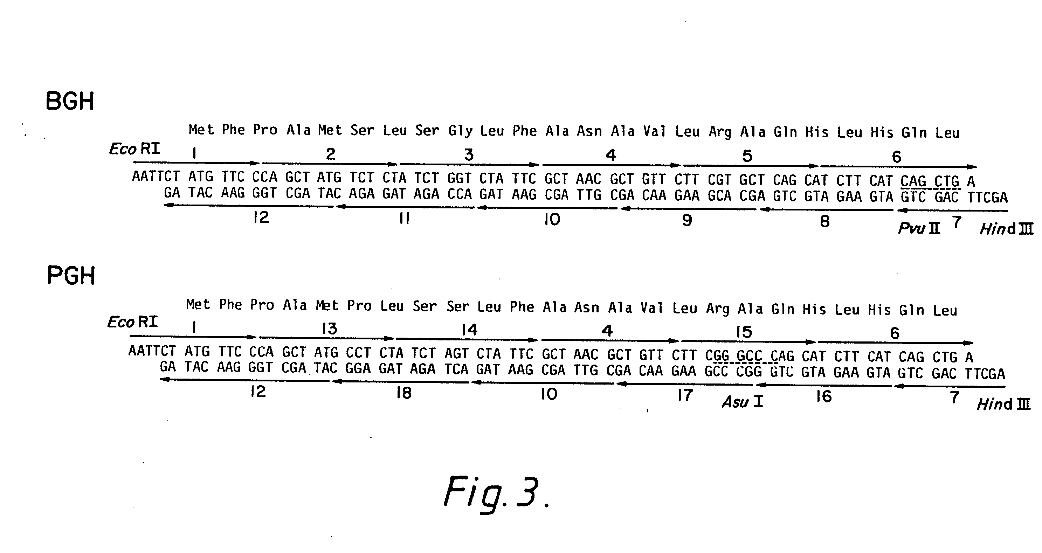

Figure 3 depicts synthetic DNA sequences coding for the N-termini of BGH and PGH. Numbers refer to individually synthesized oligodeoxyribonucleotides whose respective lengths are denoted by arrows. The overlapping design guaranteed self-assembly and the cohesive ends allowed cloning in plasmid pBR322. The restriction sites marked by broken lines were used to join the synthetic DNAs to cDNA sequences.

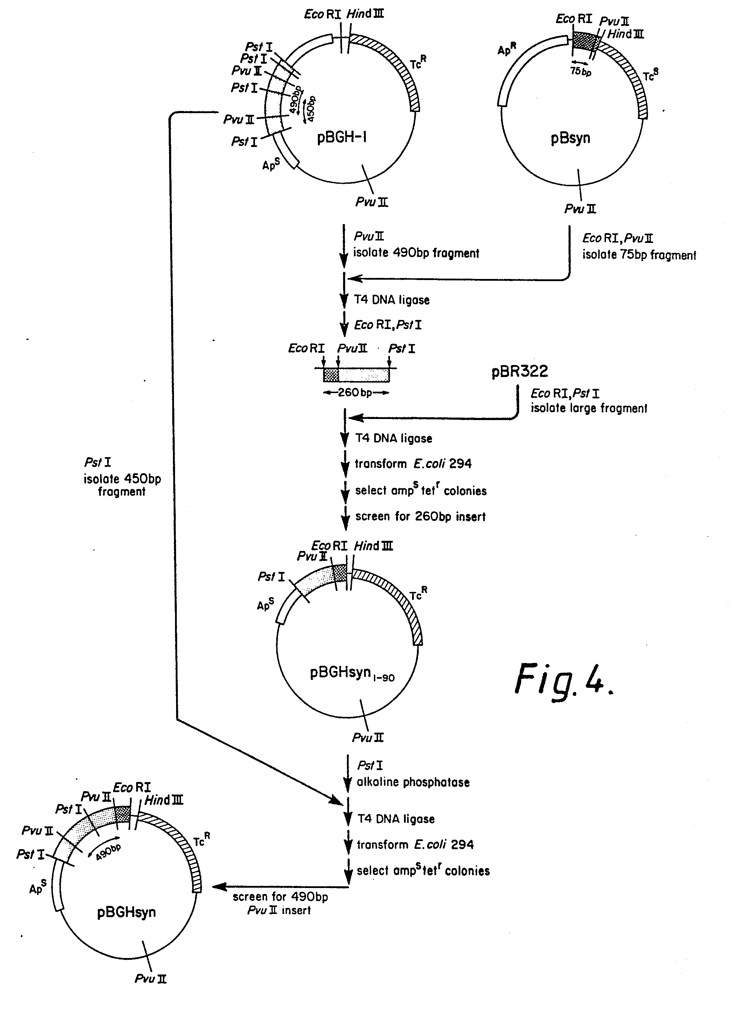

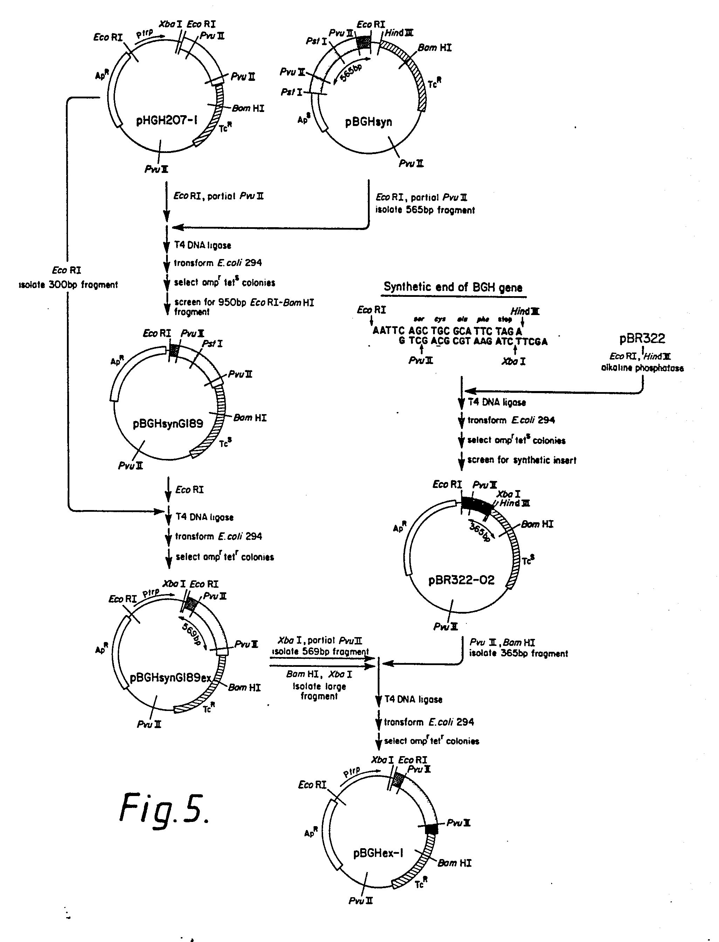

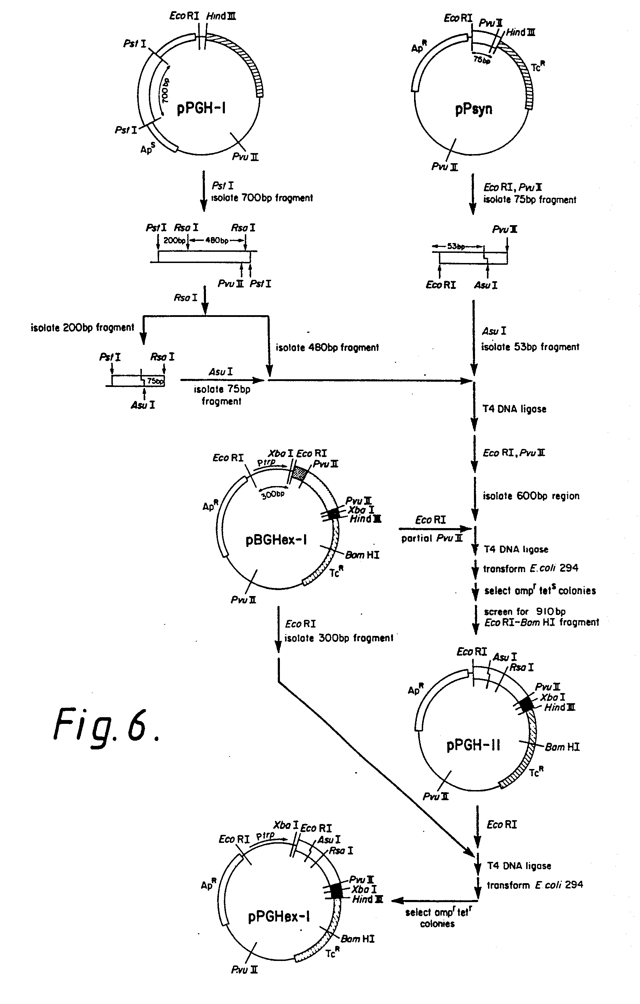

Figure 4 and 5 shows the construction strategy of a synthetic-natural hybrid gene coding for BGH, as a precursor for the construction of the expression vector of PGH - See Figure 6. pBGH-1 contains the BGH cDNA sequences (lightly stippled box) cloned into the $Ap^R$ gene in pBR322. pBsyn carries the synthetic DNA coding for a new N-terminus of BGH cloned into the EcoRI and HindIII sites of pBR322 (heavily stippled box). pBGHsyn1-90 contains the 5' half (coding for amino acids 1 to 90) of the newly constructed hybrid gene, and pBGHsyn carries the complete gene. Arrows show 5' → 3' direction of coding sequences.

pHGH207-1 carries a gene for the bacterial expression of mature HGH (4,19,39) complete with a promoter Ptrp. In plasmids pBGHsynG189 and pBGHsynG189ex the region between the EcoRI site and the distal PvuII site in the HGH gene is replaced with the homologous DNA piece from pBGHsyn (see Fig. 4). pBR322-02 contains a synthetic DNA piece (black box) coding for the correct C-terminus of BGH. This DNA piece is incorporated into pBGHsynG189ex to yield pBGHex-1 containing a functional gene for the bacterial production of BGH. Arrows show 5' → 3' direction of coding sequences. (Plasmids pBGHsynG 189ex and pBGHex-1 initially were respectively referred to internally as pBGH33-3 and pBGH33-4.)

Figure 6 depicts the construction strategy for a bacterial expression vector for PGH. pPGH-1 contains a cDNA fragment (see Fig. 1) coding for the mature PGH and 20 amino acid residues of the signal sequence, cloned into the PstI site of the Ap$^R$ gene in plasmid pBR322. pPsyn carries the synthetic DNA coding for the new front end of the bacterial PGH gene. The cloned synthetic DNA and cDNA were assembled appropriately and replace the homologous DNA in pBGHex-1 to yield plasmid pPGH-11. pPGH-11 was converted into the bacterial production vector pPGHex-1 by introducing the promoter region PTrp on a 300 bp EcoRI fragment from pBGHex-1. Arrows show 5' → 3' direction of coding sequences.

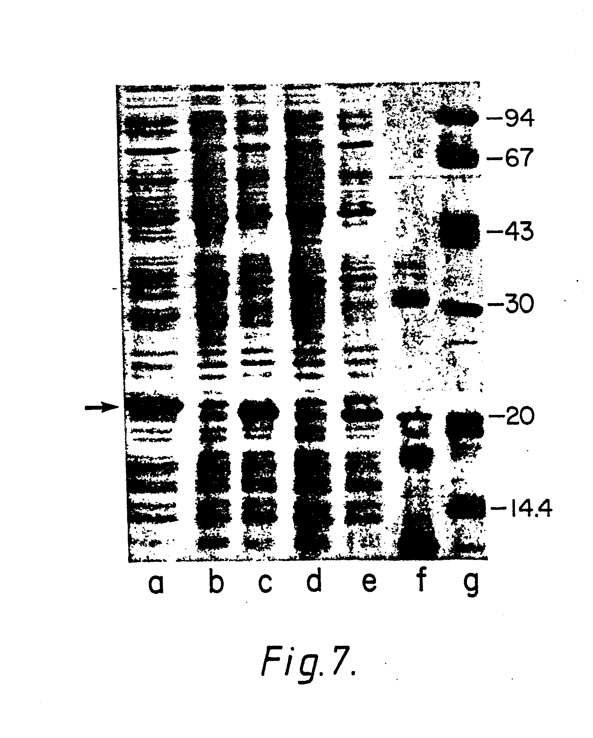

Figure 7 shows the results of bacterial protein production directed by vectors pBGHex-1 and pPGHex-1. Total protein lysates from E. coli were electrophoresed on a 12.5 percent SDS-polyacrylamide slab gel. Bacteria were transformed with vectors pBGHex-1 (lanes a,b), pPGHex-1 (lanes c,d) and pHGH207-1 (lane e). Transcription from trp promoter was induced by tryptophan starvation of bacteria (lanes a, c, e). Lanes f and g contain molecular size standards. Arrow points to 22,000 dalton proteins expressed under trp control by bacterial expression vectors for BGH, PGH and HGH.

Detailed Description

Materials. Restriction endonucleases were obtained from Bethesda Research Laboratories, DNA polymerase I (large fragment) was from Boehringer Mannheim, T4 DNA ligase from New England Biolabs, and T4 polynucleotide kinase from PL-Biochemicals. [α-$^{32}$P]dCTP and [γ-$^{32}$P]ATP were purchased from Amersham.

0200L

Preparation of RNA and cloning of cDNA. Total RNA from porcine pituitaries (obtained from local slaughterhouses) was isolated by a published procedure involving the use of guanidinium thiocyanate (24). Polyadenylated RNA was obtained from total RNA by chromatography on oligo-dT cellulose. Double-stranded cDNA was prepared from this RNA and a size fraction of the cDNA (approximately 600-1000 bp) was cloned by standard methods in E. coli 294 using plasmid pBR322 and the homopolymer annealing method as described (5).

Screening of transformants and characterization of cloned PGH cDNAs. Colonies transformed with porcine cDNA-containing plasmids were transferred to 10 96-well microtiter dishes and stamped in ordered arrays onto nitrocellulose filters. Filters containing grown colonies were processed for hybridization according to the procedure described by Grunstein and Hogness (25). Cloned cDNA from rat GH mRNA (26) was labeled to a specific activity of ~$10^8$ cpm per µg using [α-$^{32}$P]dCTP and DNA polymerase I, large fragment (27) and used as probes ($10^6$ cpm per filter) for detecting cloned PGH cDNAs. Hybridizing colonies were grown (5 ml), plasmid DNAs extracted, and cloned sequences characterized by cleavage with restriction endonucleases followed by electrophoresis of DNA fragments on 8 percent polyacrylamide gels.

DNA sequence analysis. The cloned cDNAs from plasmids pPGH-1 and pPGH-2 were isolated on polyacrylamide gels and sequenced by the dideoxy method (28,29) using primed DNA synthesis on recombinant phage M13 cloning vectors mp8 and mp9 (30) as described (31).

Assembly, molecular cloning and analysis of chemically synthesized DNA. To construct the 5' end of the bacterial gene for expressing PGH, twelve oligonucleotides had to be synthesized (see Fig. 3). (32) Aliquots (0.01 OD$_{260}$) of these oligonucleotides were phosphorylated at 37°C using T4 polynucleotide kinase, and ligated to each other at 4°C overnight, in 200 µl of 50 mM Tris-HCl

-13-

(pH 7.5), 10 mM $MgCl_2$, 10 mM dithiothreitol, and 0.5 mM ATP using 10 units of T4 DNA ligase. The ligation products were cleaved with an excess of endonucleases EcoRI and HindIII and cleavage products electrophoresed on a 10 percent polyacrylamide gel. DNA migrating at ~80 bp was sliced from the gel, eluted, and cloned into plasmid pBR322 cleaved with the same endonucleases. The new 3' terminal sequence for the bacterial PGH gene was obtained by cloning two complementary oligonucleotides 21 nucleotides long (see Fig. 5) into plasmid pBR322. Cloned synthetic DNAs were cleaved from plasmid DNA and sequenced as described above.

Construction of the bacterial expression vectors for PGH. A series of DNA fragments were transferred to different plasmids for the construction of the final expression vector pPGHex-1. All steps are diagrammed in Figs. 4-6. Generally, DNA fragments were isolated by gel electrophoresis and ligated to other fragments, or plasmid DNAs, in 20 to 50 μl of 50 mM Tris-HCl (pH 7.5), 10 mM $MgCl_2$, 0.1 mM EDTA, 10 mM dithiothreitol, and 0.5 mM ATP using 2 units of T4 DNA ligase. If partial digestions of DNAs with restriction endonucleases were required, optimal cleavage times were established experimentally.

Identification of PGH produced in bacteria. Bacterial cultures containing plasmid pPGHex-1 were grown overnight in LB broth. Aliquots were diluted 1:100 into M9 medium (for induction of Trp promoter) or LB broth (for negative control) and grown in shaker flasks to an $OD_{600}$ of 1.0. Cell pellets from 1 ml aliquots of these cultures were lysed in 2 percent SDS, 1 percent β-mercaptoethanol, and protein was precipitated with 10 volumes of cold acetone. Precipitated protein was redissolved in SDS sample buffer and aliquots electrophoresed on 12.5 percent SDS-polyacrylamide gels (33). For radioimmunoassays similar cell pellets were sonicated and protein extracts assayed for the presence of PGH.

-14-

Cloning and sequence analysis of PGH cDNA. Polyadenylated RNA prepared from porcine pituitaries was converted enzymatically into double-stranded cDNA. A 600-1,000 base pair size fraction of both DNAs was inserted into the PstI site of plasmid pBR322 DNA and cloned into E. coli using standard techniques (5). Approximately 1,000 colonies from each cDNA bank were grown in microtiter wells and aliquots of the cultures were stamped in ordered arrays onto nitrocellulose filters and processed for in situ colony hybridization (25). Colonies containing cloned growth hormone cDNA sequences were identified by making use of the known evolutionary conservation of these sequences (34). Radiolabelled cloned rat growth hormone cDNA hybridized to 12 colonies in the bovine bank and to four colonies in the porcine bank. Plasmid DNAs were prepared from the corresponding cultures and analyzed by cleavage with several restriction endonucleases followed by polyacrylamide gel electrophoresis. Analysis revealed that, for the putative PGH cDNA clones, the two with the largest inserts, pPGH-1 and -2 (both approximately 700 base pairs), were found to overlap by approximately 500 base pairs. Four of the putative BGH cDNA clones carried inserts larger than 800 bp, suggesting full-length cDNA complements. They contained two internal PstI sites, two PvuII sites and one SmaI site. The restriction maps of these two clones are shown in Fig. 1.

DNA sequence analysis of the cloned cDNAs was performed by the chain termination method (28) on single-stranded DNA templates using bacteriophage M13 vectors (30,31). The results showed that the cloned cDNAs indeed originated from porcine growth hormone mRNAs. The aligned primary structures of the cDNAs and the amino acid sequences of pre-PGH with most of its signal sequence as deduced from the nucleotide sequences are shown in Fig. 2. A large portion of the amino acid sequence of PGH was unknown (35) and is presented here for the first time. PGH differs from BGH in 18 positions (see Fig. 2). Both mature hormones start with phenylalanine although N-terminal heterogeneity is known to occur in BGH where an alanine

0200L

residue is found to precede the phenylalanine in some of the pituitary growth hormone (36). It has been suggested that this heterogeneity reflects differential removal of the signal peptide rather than genetic polymorphism (37). This notion is consistent with the amino acid sequence shown in Fig. 2 where an alanine (residue -1) precedes the phenylalanine (residue +1). Furthermore, the alanine is preceded by a glycine and the removal of signal peptides is known to occur preferentially adjacent to amino acid residues with small hydrophobic side chains (38).

Construction of expression vectors. The scheme devised to achieve efficient bacterial production of PGH is analogous to one we have used for the production of HGH (5). It employs the construction of a hybrid gene in which the section coding for the N-terminal portion of the hormones is made synthetically. Such an approach allows for the direct expression of the mature hormones by introducing an ATG initiation codon for protein synthesis in front of the triplet coding for the first amino acid.

The synthetic double-stranded DNA was assembled from 12 single-stranded oligodeoxynucleotides 11-16 bases long, 6 for each strand of the final product. The DNAs contained 24 codons which were chosen as close in sequence to the synthetic HGH codons (5) as the amino acid sequences and the recognition sites for restriction endonucleases used in assembling the hybrid genes allowed. To facilitate their insertion into plasmid pBR322 DNA the synthetic parts of the two genes were designed to contain 4 base single-stranded extensions on their 5' ends corresponding in sequence to the cohesive ends generated by endonucleases EcoRI and HindIII.

The ligation products of the two sets of 12 phosphorylated oligodeoxynucleotides were cleaved with EcoRI and HindIII and the desired ligation products were isolated from a polyacrylamide gel as bands of approximately 80 base pairs. This DNA was then inserted

-16-

into appropriately cleaved pBR322 and the cloned synthetic DNAs were analyzed by sequencing to ensure correctness.

The experimental steps used for constructing the precursor BGH hybrid gene and expression vectors are shown in Figs. 4 and 5 respectively. First, the region from pBGH-1 coding for the signal peptide plus the first 22 amino acids of the mature hormone was substituted with the synthetic part of the gene shown in Fig. 3. Briefly, the 490 base pair PvuII fragment carrying the coding sequences for BGH amino acids 23-186 was isolated and ligated to the synthetic cloned EcoRI-PvuII fragment isolated from pBsyn and containing sequences coding for the first 22 amino acids plus an N-terminal methionine. The ligation product was cleaved with endonucleases EcoRI and PstI and a 260 base pair DNA fragment was inserted into pBR322 linearized by the same two endonucleases, to yield pBGHsyn$_{1-90}$. To complete the hybrid gene construction the 450 base pair PstI fragment from pBGH-1 containing the coding sequences for BGH residues 91 to 190 (plus the 3' untranslated portion of BGH cDNA) was inserted into the single PstI site of pBGHsyn$_{1-90}$. Plasmid pBGHsyn was identified by its restriction map.

A BGH expression vector closely resembling one we have used to produce HGH in E. coli (5,39) was constructed in the hope of obtaining comparable high efficiency expression. In plasmid pHGH207-1 HGH expression is under the control of the E. coli trp promoter and transcription is in the direction of the TET$^R$ gene of pBR322 (39). The construction of the BGH expression vector was initiated by moving the 565 base pair EcoRI-PvuII (partial) DNA fragment from pBGHsyn into pHGH207-1 from which the homologous HGH hybrid gene section (and the trp promoter region) had been removed (see Fig. 5, top). The promoter region carried on a 300 base pair EcoRI fragment was then transferred into the resulting plasmid pBGHsynG189. The protein whose synthesis is directed by this plasmid is expected to contain a glycine as the penultimate amino

0200L

acid instead of the alanine residue found in this position of BGH (see Fig. 2), since the end of the hybrid gene in pBGHsynG189 (downstream from the PvuII site at amino acid codons 187-188) derives from pHGH207-1. To express the correct protein a piece of synthetic DNA encoding the C terminus of BGH was cloned into pBR322 (see Fig. 5). The final expression plasmid pBGHex-1 was assembled from this cloned synthetic DNA and two DNA fragments derived from pBGHsynG189 as shown at the bottom of Fig. 5.

The construction of the PGH expression vector is outlined in Fig. 6. Due to the absence of a PvuII site in the region coding for amino acid residues 22 and 23 of PGH the synthetic 5' part of the PGH hybrid gene was joined to the coding sequences derived from the cloned cDNA through an AsuI site which occurs at codons 16 and 17 in pPGH-1. An AsuI site was also incorporated at the corresponding position in the synthetic DNA coding for the PGH 5' region (see Fig. 3). Because of the presence of additional AsuI sites in the cloned cDNA the PGH hybrid gene was assembled from three DNA fragments (see Fig. 6).

The 700 base pair PstI fragment isolated from pPGH-1 was cleaved with endo RsaI and the resulting 200 base pair PstI-RsaI fragment was further digested with AsuI. The hybrid gene was constructed by ligating together the cloned synthetic EcoRI-AsuI 53 base pair fragment, the 75 base pair AsuI-RsaI fragment, and the 480 base pair RsaI-RsaI fragment. The product of this three-part ligation was cleaved with EcoRI and PvuII, and DNA corresponding to 600 pairs in size was isolated from a polyacrylamide gel. This DNA was then inserted into pBGHex-1, from which the EcoRI-PvuII fragment of the BGH hybrid gene had been removed, to yield pPGH-11. This plasmid contains the correct coding sequence for PGH since PGH and BGH have the same C-terminal amino acid sequence. The expression vector was obtained from pPGH-11 by inserting the 300 base pair EcoRI fragment which carries the trp promoter into this plasmid.

Bacterial production of PGH. The newly constructed expression vector was tested for its ability to direct the bacterial production of PGH. Bacterial cultures transformed with plasmid pPGHex-1 were grown under conditions known to induce trp promoter-directed expression. Fig. 7 shows protein extracts from such cultures resolved on SDS-polyacrylamide gels. A prominent band corresponding to a protein of about 22,000 daltons is visible; this band is absent from extracts of bacteria grown under conditions where transcription from the trp promoter is shut off. The appearance of the 22,000 dalton protein correlates with positive RIA data for BGH and PGH indicating that these proteins are indeed the pituitary growth hormones. Pilot studies using high density fermentation show that the expression vector of pPGHex-1 promotes a yield of approximately 1.5g of PGH per liter of bacterial culture.

0200L

0111389

-19-

Bibliography

1. British Patent Application Publn. No. 2055382A.

2. German Offenlegungsschrift 2644432.

3. Chang et al., Nature 275, 617 (1978).

4. Itakura et al., Science 198, 1056 (1977).

5. Goeddel et al., Nature 281, 544 (1979).

6. Goeddel et al., Nucleic Acids Research 8, 4057 (1980).

7. European Patent Application Publn. No. 0036776.

8. Siebenlist et al., Cell 20, 269 (1980).

9. Stinchcomb et al., Nature 282, 39 (1979).

10. Kingsman et al., Gene 7, 141 (1979).

11. Tschumper et al., Gene 10, 157 (1980).

12. Mortimer et al., Microbiological Reviews 44, 519 (198 ).

13. Miozzari et al., Journal of Bacteriology 134, 48 (1978).

14. Jones, Genetics 85, 23 (1977).

15. Hitzeman, et al., J. Biol. Chem. 255, 12073 (1980).

16. Tissue Culture, Academic Press, Kruse and Patterson eds, (1973).

17. Gluzman, Cell 23, 175 (1981).

18. Bolivar et al., Gene 2, 95 (1977).

19. Lusky et al., Nature 293, 79 (1981).

20. Gluzman et al., Cold Spring Harbor Symp. Quant. Biol. 44, 293 (1980).

21. The Molecular Biology of Yeast (Aug 11-18, 1981), Cold Spring Harbor Laboratory, Cold Spring Harbor, New York.

22. Fiers et al., Nature 273, 113 (1978).

23. Reddy et al., Science 200, 494 (1978).

24. Ullrich, A., et al. Science 196, 1313 (1977).

0200L

25. Grunstein, M., and Hogness, D.S. Proc.Natl.Acad.Sci.(U.S.A.) 72, 3961 (1975).

26. Seeburg, P.H., et al. Nature 270, 486 (1977).

27. Fiddes, J.C., et al., Proc.Natl.Acad.Sci. (U.S.A.) 76, 4294 (1979).

28. Sanger, F., et al. Proc.Natl.Acad.Sci. (U.S.A.) 74, 5463 (1977).

29. Sanger, F., and Coulson, A.R. FEBS Letters 87, 107 (1980).

30. Messing, J. and Vieira, J. Gene 19, 269 (1982).

31. Messing, J., et al., Nucleic Acids Res. 9, 309 (1981).

32. Crea, R., and Horn, T. Nucleic Acids Res. 8, 2331 (1980).

33. Laemmli, U.K. Nature 227, 680 (1970).

34. Shine, J., et al., Nature 270, 494 (1977).

35. Mills, J.B., et al., J. Biol. Chem. 245, 3407 (1970).

36. Li, C.H., and Ash, L. (1953) J. Biol. Chem. 203, 419 (1953).

37. Wallis, M., and Davies, R.V. In Growth Hormone and Related Peptides (Pecile, A., and Muller, E.E., eds.). Proc. 3rd Internat. Symposium, Milan, pp. 1-13. Excerpta Medica American Elsevier (1976).

38. Lingappa, V.R., and Blobel, G. Recent Prog. Horm. Res. 36, 451 (1980).

39. De Boer, H.A., et al. In Promoters, Structure and Function. Praeger Scientific Publishing Co. (Chamberlin, M.J., and Rodriguez, R., eds.) 1982.

-21-

CLAIMS

1. Substantially pure porcine growth hormone whose amino acid sequence is substantially as is shown in Figure 2 hereof.

2. Porcine growth hormone according to Claim 1, as a product of genetically altered cell culture.

3. Porcine growth hormone according to Claim 1, unaccompanied by associated native glycosylation.

4. A DNA sequence comprising a sequence encoding porcine growth hormone substantially as is shown in Figure 2 hereof.

5. The DNA sequence according to Claim 4 operably linked with a DNA sequence capable of effecting expression thereof.

6. An expression vehicle capable, in a transfected cell culture, of expressing a DNA sequence according to Claim 5.

7. A cell culture transfected with the vehicle according to Claim 6.

8. A microorganism according to Claim 7, obtained by transfecting an E. coli strain.

9. A cell culture according to Claim 7, obtained by transfecting a mammalian cell culture.

10. Plasmid pPGHex-1.

11. A cell culture transfected with the plasmid according to Claim 10.

0200L

12. A culture of cells genetically altered so as to direct the production of porcine growth hormone.

13. A composition of matter comprising porcine growth hormone essentially free of other proteins of porcine origin.

0200L

Fig.1.

```
                                                    CAGGGTCCTGTGGACAGCTCACCAGCT ATG ATG
BGH                                                                         met met
```

```
        GCT GCA GGC CCC CGG ACC TCC CTG CTC CTG GCT TTC GCC CTG CTC TGC CTG CCC TGG ACT CAG GTG GTG GGC GCC
BGH     ala ala gly pro arg thr ser leu leu leu ala pne ala leu leu cys leu pro trp thr gln val val gly ala
```

```
                ACC TCC GTG CTC CTG GCT TTC GCC CTG CTC TGC CTG CCC TGG ACT CAG GAG GTG GGA GCC
PGH             thr ser val leu leu ala pne ala leu leu cys leu pro trp thr gln glu val gly ala
                -20                                            -10                         -1
```

```
        TTC CCA GCC ATG TCC TTG TCC GGC CTG TTT GCC AAC GCT GTG CTC CGG GCT CAG CAC CTG CAT CAG CTG GCT GCT
BGH     phe pro ala met ser leu ser gly leu pne ala asn ala val leu arg ala gln his leu his gln leu ala ala
```

```
        TTC CCA GCC ATG CCC TTG TCC AGC CTA TTT GCC AAC GCC GTG CTC CGG GCC CAG CAC CTG CAC CAA CTG GCT GCC
PGH     phe pro ala met pro leu ser ser leu phe ala asn ala val leu arg ala gln his leu his gln leu ala ala
        1                       10                                            20
```

```
        GAC ACC TTC AAA GAG TTT GAG CGC ACC TAC ATC CCG GAG GGA CAG AGA TAC TCC ATC CAG AAC ACC CAG GTT GCC
BGH     asp thr phe lys glu phe glu arg thr tyr ile pro glu gly gln arg tyr ser ile gln asn thr gln val ala
```

```
        GAC ACC TAC AAG GAG TTT GAG CGC GCC TAC ATC CCG GAG GGA CAG AGG TAC TCC ATC CAG AAC GCC CAG GCT GCC
PGH     asp thr tyr lys glu pne glu arg ala tyr ile pro glu gly gln arg tyr ser ile gln asn ala gln ala ala
                        30                                            40                         50
```

```
        TTC TGC TTC TCT GAA ACC ATC CCG GCC CCC ACG GGC AAG AAT GAG GCC CAG CAG AAA TCA GAC TTG GAG CTG CTT
BGH     phe cys phe ser glu thr ile pro ala pro thr gly lys asn glu ala gln gln lys ser asp leu glu leu leu
```

```
        TTC TGC TTC TCG GAG ACC ATC CCG GCC CCC ACG GGC AAG GAC GAG GCC CAG CAG AGA TCG GAC GTG GAG CTG CTG
PGH     phe cys phe ser glu thr ile pro ala pro thr gly lys asp glu ala gln gln arg ser asp val glu leu leu
                                        60                                            70
```

```
        CGC ATC TCA CTG CTC CTC ATC CAG TCG TGG CTC GGG CCC CTG CAG TTC CTC AGC AGA GTC TTC ACC AAC AGC TTG
BGH     arg ile ser leu leu leu ile gln ser trp leu gly pro leu gln phe leu ser arg val phe thr asn ser leu
```

```
        CGC TTC TCG CTG CTG CTC ATC CAG TCG TGG CTC GGG CCC GTG CAG TTC CTC AGC AGG GTC TTC ACC AAC AGC CTG
PGH     arg phe ser leu leu leu ile gln ser trp leu gly pro val gln phe leu ser arg val phe thr asn ser leu
                        80                                            90                         100
```

```
        GTG TTT GGC ACC TCG GAC CGT GTC TAT GAG AAG CTG AAG GAC CTG GAG GAA GGC ATC CTG GCC CTG ATG CGG GAG
BGH     val phe gly thr ser asp arg val tyr glu lys leu lys asp leu glu glu gly ile leu ala leu met arg glu
```

```
        GTG TTT GGC ACC TCA GAC CGC GTC TAC GAG AAG CTG AAG GAC CTG GAG GAG GGC ATC CAG GCC CTG ATG CGG GAG
PGH     val phe gly thr ser asp arg val tyr glu lys leu lys asp leu glu glu gly ile gln ala leu met arg glu
                                        110                                           120
```

```
        CTG GAA GAT GGC ACC CCC CGG GCT GGG CAG ATC CTC AAG CAG ACC TAT GAC AAA TTT GAC ACA AAC ATG CGC AGT
BGH     leu glu asp gly thr pro arg ala gly gln ile leu lys gln thr tyr asp lys phe asp thr asn met arg ser
```

```
        CTG GAA GAT GGC AGC CCC CGG GCA GGA CAG ATC CTC AAG CAA ACC TAC GAC AAA TTT GAC ACA AAC TTG CGC AGT
PGH     leu glu asp gly ser pro arg ala gly gln ile leu lys gln thr tyr asp lys phe asp thr asn leu arg ser
                        130                                           140                        150
```

```
        GAC GAC GCG CTG CTC AAG AAC TAC GGT CTG CTC TCC TGC TTC CGG AAG GAC CTG CAT AAG ACG GAG ACG TAC CTG
BGH     asp asp ala leu leu lys asn tyr gly leu leu ser cys phe arg lys asp leu his lys thr glu thr tyr leu
```

```
        GAT GAC GCG CTG CTT AAG AAC TAC GGG CTG CTC TCC TGC TTC AAG AAG GAC CTG CAC AAG GCT GAG ACA TAC CTG
PGH     asp asp ala leu leu lys asn tyr gly leu leu ser cys phe lys lys asp leu his lys ala glu thr tyr leu
                                        160                                           170
```

```
        AGG GTC ATG AAG TGC CGC CGC TTC GGG GAG GCC AGC TGC GCC TTC TAGTTGCCAGCCATCTGTTGTTTGCCCCTCCCCCGTGCC
BGH     arg val met lys cys arg arg phe gly glu ala ser cys ala phe
```

```
        CGG GTC ATG AAG TGT CGC CGC TTC GTG GAG AGC AGC TGT GCC TTC TAGTTGCTGGGCATCTCTGTTGCCCCTCCCCAGTACCTC
PGH     arg val met lys cys arg arg phe val glu ser ser cys ala phe
                        180                                   190
```

```
        TTCCTTGACCCTGGAAGGTGCCACTCCCACTGTCCTTTCCTAATAAAATGAGGAAATTGCATCGCA

        CCCTGGTGCCCTGGAAAGTGCCACCCCAATGCCTGCTGTCCTTTCCTAATAAAACCAGGTTGCATCGTA
```

*Fig. 2.*

**BGH**

```
                Met Phe Pro Ala Met Ser Leu Ser Gly Leu Phe Ala Asn Ala Val Leu Arg Ala Gln His Leu His Gln Leu
Eco RI      1                2               3               4               5               6
        AATTCT ATG TTC CCA GCT ATG TCT CTA TCT GGT CTA TTC GCT AAC GCT GTT CTT CGT GCT CAG CAT CTT CAT CAG CTG A
        GA TAC AAG GGT CGA TAC AGA GAT AGA CCA GAT AAG CGA TTG CGA CAA GAA GCA CGA GTC GTA GAA GTA GTC GAC TTCGA
                12              11              10              9               8           PvuⅡ  7   HindⅢ
```

**PGH**

```
                Met Phe Pro Ala Met Pro Leu Ser Ser Leu Phe Ala Asn Ala Val Leu Arg Ala Gln His Leu His Gln Leu
Eco RI      1                13              14              4               15              6
        AATTCT ATG TTC CCA GCT ATG CCT CTA TCT AGT CTA TTC GCT AAC GCT GTT CTT CGG GCC CAG CAT CTT CAT CAG CTG A
        GA TAC AAG GGT CGA TAC GGA GAT AGA TCA GAT AAG CGA TTG CGA CAA GAA GCC CGG GTC GTA GAA GTA GTC GAC TTCGA
                12              18              10              17   AsuⅠ     16              7   HindⅢ
```

*Fig.3.*

Fig.4.

Fig.5.

Fig. 6.

Fig.7.